# EUROPEAN PATENT SPECIFICATION

(11) **EP 3 421 995 B1**
(45) Date of publication and mention of the grant of the patent: **31.08.2022**
(21) Application number: 17756556.1
(22) Date of filing: 22.02.2017
(51) Int. Cl.: G01N 33/53, G01N 33/543, G01N 33/558

(54) **IMMUNOCHROMATOGRAPHY ANALYSIS DEVICE FOR DETECTING GLIADIN, IMMUNOCHROMATROGRAPHY ANALYSIS KIT AND IMMUNOCHROMATOGRAPHY ANALYSIS METHOD**
IMMUNCHROMATOGRAFISCHE ANALYSEVORRICHTUNG ZUM NACHWEIS VON GLIADIN, IMMUNCHROMATROGRAFISCHES ANALYSEKIT UND IMMUNCHROMATOGRAFISCHES ANALYSEVERFAHREN
DISPOSITIF D'ANALYSE D'IMMUNOCHROMATOGRAPHIE POUR DÉTECTER LA GLIADINE, KIT D'ANALYSE D'IMMUNOCHROMATOGRAPHIE ET PROCÉDÉ D'ANALYSE D'IMMUNOCHROMATOGRAPHIE

(30) Priority: 23.02.2016 JP 2016032418
(43) Date of publication of application: 02.01.2019
(73) Proprietor: Tanaka Kikinzoku Kogyo K.K., Chiyoda-ku Tokyo 100-6422 (JP)
(72) Inventor: SUZUKI Keita, Hiratsuka-shi Kanagawa 254-0076 (JP); MOCHIZUKI Hiroko, Hiratsuka-shi Kanagawa 254-0076 (JP); IWAMOTO Hisahiko, Hiratsuka-shi Kanagawa 254-0076 (JP)
(74) Representative: Hoffmann Eitle
(86) International application number: PCT/JP2017/006676
(87) International publication number: WO 2017/146118

(56) References cited:
- EP-A1- 1 731 907
- EP-A1- 2 224 239
- EP-A2- 2 660 592
- WO-A1-2016/145061
- WO-A2-2009/139887
- JP-A- 2006 512 893
- JP-A- 2011 099 789
- JP-A- 2011 099 789
- JP-B2- 5 043 073
- US-A1- 2008 152 780
- US-A1- 2008 152 780
- BELEN MORON ET AL: "Toward the Assessment of Food Toxicity for Celiac Patients: Characterization of Monoclonal Antibodies to a Main Immunogenic Gluten Peptide", PLOS ONE, vol. 3, no. 5, 28 May 2008 (2008-05-28), pages e2294-1-e2294-13, XP055074191, DOI: 10.1371/journal.pone.0002294
- "Reagents for food, agriculture and enviroment testing 2015", XEMA 2015 Product Catalogue, 15 March 2015 (2015-03-15), XP055537897, Retrieved from the Internet: URL:http://xema-medica.com/eng/agriculture /Food%20reagents%20catalogue%202015.pdf [retrieved on 2017-04-24]
- Israel Valdés ET AL: "Innovative approach to low-level gluten determination in foods using a novel sandwich enzyme-linked immunosorbent assay protocol :", European Journal of Gastroenterology and Hepatology, vol. 15, no. 5, 1 May 2003 (2003-05-01), pages 465-747, XP055685396, UK ISSN: 0954-691X, DOI: 10.1097/01.meg.0000059119.41030.df

### SEQUENCE LISTING

<110> TANAKA KIKINZOKU KOGYO K.K.
<120> IMMUNOCHROMATOGRAPHY ANALYSIS APPARATUS FOR DETECTING GLIADIN,IMMUNOCHROMATOGRAPHY ANALYSIS KIT,AND IMMUNOCHROMATOGRAPHY ANALYSIS METHOD
<130> W521720
<150> JP2016/032418
   <151> 2016-02-23
<160> 6
<170> PatentIn version 3.5
<210> 1
   <211> 292
   <212> PRT
   <213> \203¿-gliadin
<400> 1
   Met Lys Thr Phe Leu Ile Leu Ala Leu Leu Ala Ile Val Ala Thr Thr
   1               5                   10                  15
   Ala Thr Thr Ala Val Arg Val Pro Val Pro Gln Leu Gln Pro Gln Asn
               20                  25                  30
   Pro Ser Gln Gln Glu Pro Gln Glu Gln Val Pro Leu Val Gln Gln Gln
           35                  40                  45
   Gln Phe Leu Gly Gln Gln Gln Pro Phe Pro Pro Gln Gln Pro Tyr Pro
       50                  55                  60
   Gln Pro Gln Pro Phe Pro Ser Gln Gln Pro Tyr Leu Gln Leu Gln Leu
   65                  70                  75                      80
   Phe Pro Gln Pro Gln Gln Pro Tyr Ser Gln Pro Gln Pro Phe Arg Pro
                   85                  90                  95
   Gln Gln Pro His Pro Gln Pro Gln Pro Gln Tyr Ser Gln Pro Gln Gln
               100                 105                 110
   Pro Ile Ser Gln Gln Gln Gln Gln Gln Gln Gln Gln Gln Gln Gln Gln
           115                 120                 125
   Gln Gln Ile Leu Gln Gln Ile Leu Gln Gln Gln Leu Ile Pro Cys Met
       130                 135                 140
   Asp Val Val Leu Gln Gln His Asn Ile Ala His Gly Ser Ser Gln Val
   145                 150                 155                     160
   Leu Gln Gln Ser Thr Tyr Gln Leu Leu Gln Gln Leu Cys Cys Gln Gln
                   165                 170                 175
   Leu Trp Gln Ile Pro Glu Gln Ser Arg Cys Gln Ala Ile His Asn Val
               180                 185                 190
   Val His Ala Ile Ile Leu His Gln Gln Gln Gln Gln Gln Gln Gln Gln
           195                 200                 205
   Gln Gln Val Gln Gln Gln Pro Ser Ser Gln Val Ser Tyr Gln Gln Pro
       210                 215                 220
   Gln Gln Gln Tyr Pro Ser Gly Gln Gly Ser Phe Gln Pro Ser Gln Gln
   225                 230                 235                 240
   Asn Pro Gln Ala Gln Gly Phe Val Gln Pro Gln His Leu Pro Gln Leu
                   245                 250                 255
   Glu Glu Ile Arg Asn Leu Ala Leu Gln Thr Leu Pro Ala Met Cys Asn
               260                 265                 270
   Val Tyr Ile Pro Pro Tyr Cys Ser Thr Thr Ile Ala Pro Phe Gly Ile
           275                 280                 285
   Phe Gly Thr Asn
       290
<210> 2
   <211> 21
   <212> PRT
   <213> \203¿-gliadin
<400> 2
   Gln Pro Gln Asn Pro Ser Gln Gln Gln Pro Gln Glu Gln Val Pro Leu
   1               5                   10                  15
   Val Gln Gln Gln Gln
               20
<210> 3
   <211> 30
   <212> PRT
   <213> \203¿-gliadin
<400> 3
   Pro Gln Gln Pro Tyr Pro Gln Pro Gln Pro Phe Pro Ser Gln Gln Pro
   1               5                   10                  15
   Tyr Leu Gln Leu Gln Pro Phe Pro Gln Pro Gln Pro Phe Pro
               20                  25                  30
<210> 4
   <211> 30
   <212> PRT
   <213> \203¿-gliadin
<400> 4
   Pro Gln Leu Pro Tyr Pro Gln Pro Gln Ser Phe Pro Pro Gln Gln Pro
   1               5                   10                  15
   Tyr Pro Gln Gln Gln Pro Gln Tyr Leu Gln Pro Gln Gln Pro
               20                  25                  30
<210> 5
   <211> 22
   <212> PRT
   <213> \203¿-gliadin
<400> 5
   Pro Ser Ser Gln Val Ser Phe Gln Gln Pro Gln Gln Gln Tyr Pro Ser
   1               5                   10                  15
   Ser Gln Val Ser Phe Gln
               20
<210> 6
   <211> 21
   <212> PRT
   <213> \203¿-gliadin
<400> 6
   Gln Tyr Pro Ser Ser Gln Val Ser Phe Gln Pro Ser Gln Leu Asn Pro
   1               5                   10                  15
   Gln Ala Gln Gly Ser
               20

## Description

### Technical Field

The present invention relates to an immunochromatography analysis device for detecting gliadin, an immunochromatography analysis kit and an immunochromatography analysis method.

### Background Art

Increased rates of food allergies have recently become an enormous social problem. Food allergic patients cannot take foods containing the substance causing the allergy (allergen). Patients allergic to food allergens contained in foods develop various allergic symptoms such as asthma, dermatitis, gastrointestinal diseases and anaphylactic shock, which sometimes lead to a serious case. Also due to the increasing number of patients with food allergies, there is enormous consumers' interest in the food safety.

Although eggs, cow's milk and soybeans had been considered as the three most common food allergens, it has been recently found that, of the patients with atopic dermatitis caused by food allergies, the number of patients sensitive to wheat is greater than expected as compared to the number of patients sensitive to the three most common allergens. One of the substances which cause wheat allergy is gliadin. Gliadin is a glycoprotein present in wheat and is classified into four types, α, β, γ and ω.

It is desirable to restrict the intake of a food causing the allergy to prevent the onset of an allergic symptom to the food. However, wheat is not only used as staple foods such as bread, noodles and pasta but also contained as a raw material of various processed foods. Thus, a method by which the presence or absence of a substance causing wheat allergy in foods can be determined easily has been required.

The significance of an immunoassay of the strip type for an immunochromatography analysis as a simple extracorporeal diagnostic kit or a mobile diagnostic device for detecting an antigen in a sample solution using the specific reactivity of an antibody has been growing. During food inspection, it is required to test a large number of food samples in a short time, and an immunochromatography analysis method is a versatile method due to its rapidity and simplicity.

A test drug for an immunochromatography analysis method which is obtained by binding an antibody to gold nanoparticles and which is used as a labeling reagent is inexpensive and simple to use, and the test finishes in a short time. Thus, the test drug is used for detecting a specific minor component in a thin extracted sample with less noise.

As methods by which the presence or absence of a substance causing wheat allergy in foods can be determined easily, methods using the immunochromatography analysis method have been known so far. For example, Patent Literature 1 discloses a method for detecting wheat gliadin in foods using an immunochromatographic strip which uses two kinds of anti-wheat gliadin monoclonal antibodies which recognize wheat gliadin and recognize different epitopes.

### Background Art Document

### Patent Literature

Patent Literature 1: Japanese Patent No. 5043073

### Summary of Invention

### Technical Problem

However, a large amount of contaminants which cause noise, other than gliadin, are contained in a food sample. The conventional immunochromatography analysis devices for detecting gliadin cause cross-reactions with antigens in foods other than gliadin, and the specificities to gliadin are insufficient. As a result, a false positive reaction, namely a positive reaction to gliadin even in the absence of the detection target gliadin, is caused in the conventional devices.

Therefore, an object of the invention is to provide an immunochromatography analysis device for detecting gliadin in which a cross-reaction with an antigen present in a food sample other than gliadin is inhibited and which has improved specificity to gliadin as compared to those of the conventional ones, an immunochromatography analysis kit and an immunochromatography analysis method using the device or the kit.

### Solution to Problem

As described above, gliadin is a glycoprotein present in wheat and is classified into four types, α, β, γ and ω. As a result of intensive studies, the inventors of the present inevntion have found for the first time that antibodies which recognize α-gliadin of the four types of gliadin are less cross-reactive to antigens other than gliadin and can specifically detect gliadin.

The inventors have found for the first time that antibodies which recognize at least one amino acid sequence of the five amino acid sequences of SEQ ID NOs: 2 to 6 which are present in the whole amino acid sequence (SEQ ID NO: 1) of α-gliadin can especially detect gliadin more specifically.

Moreover, the inventors have found for the first time that the occurrence of false positive reactions can be reduced more when the antibodies contained in the labeling substance retaining part and in the detection part of the immunochromatography analysis device are the same antibody.

Based on the findings, the inventors have completed the invention.

Therefore, the invention is as follows.
1. An immunochromatography analysis device for detecting gliadin, including a sample addition part, a labeling substance retaining part, a chromatography medium part having a detection part and an absorption part,
   wherein the labeling substance retaining part contains a first antibody recognizing α-gliadin and the detection part contains a second antibody recognizing α-gliadin, wherein the first and second antibody are the same, and the first antibody binds to any of the repeated sequences present in α-gliadin and the second antibody binds to the same repeated sequence which is present at a different site from the site to which the first antibody bound; wherein the first and second antibody are monoclonal antibodies, and the first and second antibody recognize at least one amino acid sequence of the five amino acid sequences of SEQ ID NOs: 2 to 6 which are present in the whole amino acid sequence of α-gliadin , wherein the amount of antibody in the labelling substance retaining part is from 0.01 µg/device to 1.0 µg/ device.
2. An immunochromatography analysis kit including the immunochromatography analysis device according to the above 1 and an analyte dilution solution for diluting and developing an analyte.
5. An immunochromatography analysis method for detecting gliadin in an analyte using an immunochromatography analysis device including a sample addition part, a labeling substance retaining part, a chromatography medium part having a detection part and an absorption part, wherein the labeling substance retaining part contains a first antibody recognizing α-gliadin and the detection part contains a second antibody recognizing α-gliadin,and the first and second antibody are the same; wherein the first and second antibody are monoclonal antibodies, and the first and second antibody recognize at least one amino acid sequence of the five amino acid sequences of SEQ ID NOs: 2 to 6 which are present in the whole amino acid sequence of α-gliadin; and wherein the amount of antibody in the labelling substance retaining part is from 0.01 µg/device to 1.0 µg/ device, wherein the method includes the following steps (1) to (4):
   (1) a step of adding an analyte-containing solution obtained by diluting the analyte with an analyte dilution solution to the sample addition part,
   (2) a step of allowing the first antibody to bind to any of the repeated sequences present in α-gliadin,
   (3) a step of developing the analyte and the antibody as a mobile phase on the chromatography medium part, and
   (4) a step of detecting α-gliadin in the developed mobile phase with a second antibody that binds to the same repeated sequence which is present at a different site from the site to which the first antibody bound.

### Effects of Invention

In the invention, using antibodies which recognize especially α-gliadin of gliadins for the immunochromatography analysis device for detecting gliadin, a cross-reaction with another antigen can be inhibited, and gliadin can be detected specifically.

By using especially antibodies which recognize at least one amino acid sequence of the five amino acid sequences of SEQ ID NOs: 2 to 6 which are present in the whole amino acid sequence of α-gliadin, gliadin can be detected more specifically.

Moreover, using the same antibody as the antibodies contained in the labeling substance retaining part and in the detection part of the immunochromatography analysis device, the occurrence of a false positive reaction can be reduced more.

### Brief Description of Drawings

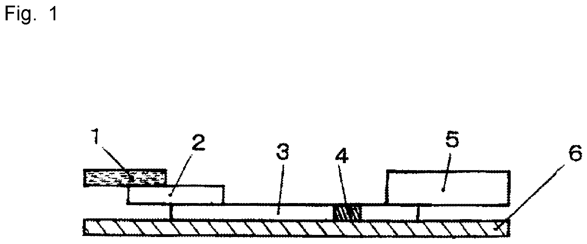

[Fig. 1] Fig. 1 is a cross section for explaining the structure of the immunochromatography analysis device of an embodiment of the invention.

### Description of Embodiments

Embodiments for carrying out the invention are explained below.

### <Immunochromatography Analysis Device>

The immunochromatography analysis device for detecting gliadin of the invention has a sample addition part to which an analyte sample is added, a labeling substance retaining part holding a labeling substance, a chromatography medium part having a detection part for detecting gliadin and an absorption part for absorbing a liquid which has passed through the detection part, and the device is characterized in that the labeling substance retaining part and the detection part contain an antibody recognizing α-gliadin.

The antibodies used for the immunochromatography analysis device of the invention are antibodies which recognize α-gliadin.

Gliadin refers to wheat flour proteins which dissolve in 70% ethanol and is a mixture of many kinds of proteins which have similar molecular weights and similar amino acid compositions. Gliadin is classified into four types, α-, β-, γ- and ω- gliadins, based on the mobilities in electrophoresis.

Moreover, gliadin is classified into three groups by gel filtration using a Sephadex G-100 column. The three groups are low molecular weight gliadins having molecular weights of about 30,000 (α, β and γ-gliadins), high molecular weight gliadins having molecular weights of about 100,000 and ω-gliadin having a molecular weight of about 70,000.

Of these, α-gliadin is a low molecular weight gliadin which is composed of the 292 amino acids of SEQ ID NO: 1 and which has a molecular weight of about 30,000.

In the invention, using antibodies which recognize especially α-gliadin of various gliadins for the immunochromatography analysis device, a cross-reaction with an antigen other than gliadin can be inhibited, and gliadin can be detected specifically.

In particular, of antibodies which recognize α-gliadin, antibodies which recognize at least one amino acid sequence of the five amino acid sequences of SEQ ID NOs: 2 to 6 which are present in the whole amino acid sequence of α-gliadin are used in the present invention.

Of the antibodies, antibodies which recognize the amino acid sequence of SEQ ID NO: 3 are especially preferable because many repeated sequences described below are included.

When an antibody which recognizes at least one of the amino acid sequences is used, a cross-reaction with another antigen can be reduced more, and gliadin can be detected more specifically.

The amino acid sequence of SEQ ID NO: 2 (QPQNPSQQQPQEQVPLVQQQQ) indicates the amino acid sequence of from the 29th to 49th residues of the whole amino acid sequence of α-gliadin.

The amino acid sequence of SEQ ID NO: 3 (PQQPYPQPQPFPSQQPYLQLQPFPQPQPFP) indicates the amino acid sequence of from the 59th to 88th residues of the whole amino acid sequence of α-gliadin.

The amino acid sequence of SEQ ID NO: 4 (PQLPYPQPQSFPPQQPYPQQQPQYLQPQQP) indicates the amino acid sequence of from the 89th to 118th residues of the whole amino acid sequence of α-gliadin.

The amino acid sequence of SEQ ID NO: 5 (PSSQVSFQQPQQQYPSSQVSFQ) indicates the amino acid sequence of from the 236th to 257th residues of the whole amino acid sequence of α-gliadin.

The amino acid sequence of SEQ ID NO: 6 (QYPSSQVSFQPSQLNPQAQGS) indicates the amino acid sequence of from the 248th to 268th residues of the whole amino acid sequence of α-gliadin.

Antibodies used in the invention are monoclonal antibodies.

As the antibodies which recognize α-gliadin, i. e. as the antibodies which recognize at least one amino acid sequence of the five amino acid sequences of SEQ ID NOs: 2 to 6, XGY06 and XGY10 (manufactured by XEMA, product names: Murine monoclonal antibodies to prolaminsclone Nos. XGY06 and XGY10), which were confirmed to recognize the above sequences in the Examples below, and the like can be purchased and used.

Next, the immunochromatography analysis device of the invention is explained referring to the drawing. In this description, "immobilize" means that an antibody is arranged on a carrier such as a membrane in a manner that the antibody does not move, and "hold" means that an antibody is arranged in a manner that the antibody can move in a carrier such as a membrane or on the surface thereof.

The immunochromatography analysis device of the invention is composed of a sample addition part (1), a labeling substance retaining part (2), a chromatography medium part (3), a detection part (4), an absorption part (5) and a backing sheet (6) as shown in Fig. 1.

The sample addition part (1) is a part in the immunochromatography analysis device to which an analyte sample is added. The sample addition part (1) can be composed of a porous sheet having the properties of rapidly absorbing the analyte sample but allowing the analyte sample to move rapidly with weak holding power. Examples of the porous sheet include cellulose filter paper, glass fibers, polyurethane, polyacetate, cellulose acetate, nylon, cotton cloth and the like.

The labeling substance retaining part (2) contains a labeling substance described below, and the labeling substance is held in the labeling substance retaining part (2) in the form of a labeling reagent in which the labeling substance is bound to the antibody which recognizes α-gliadin. When an analyte moves in the labeling substance retaining part (2), the labeling reagent binds to gliadin in the analyte. For the labeling substance retaining part (2), a membrane of glass fibers or cellulose is usually used.

The amount of the antibody recognizing α-gliadin in the labeling substance retaining part (2) (also called the first antibody below) is, according to the invention, from 0.01 µg/device to 1.0 µg/device, preferably from 0.05 µg/device to 0.75 µg/device, and more preferably from 0.075 µg/device to 0.5 µg/device.

The amount of the first antibody per unit area of the labeling substance retaining part (2) is usually from 0.006 µg/cm² to 0.42 µg/cm², preferably from 0.01 µg/cm² to 0.3 µg/cm², and more preferably from 0.01 µg/cm² to 0.2 µg/cm².

An enzyme or the like is also generally used for labeling the detection reagent in an immunochromatography analysis, but an insoluble carrier is preferably used as the labeling substance because the insoluble carrier is suitable for visually determining the presence of the substance to be detected. A labeled detection reagent can be prepared by binding the antibody which recognizes α-gliadin (the first antibody) to an insoluble carrier. In this regard, as a method for binding the antibody which recognizes α-gliadin (the first antibody) to the insoluble carrier, a known method can be applied.

As the insoluble carrier used as the labeling substance, particles of a metal such as gold, silver or platinum, particles of a metal oxide such as iron oxide, particles of a nonmetal such as sulfur, latex particles of a synthetic polymer or other carriers can be used.

The insoluble carrier is a labeling substance which is suitable for visually determining the presence of the substance to be detected and preferably has a color to make the visual determination easy. Metal particles and metal oxide particles themselves have peculiar natural colors according to the particle diameter, and the colors can be used as labels.

The insoluble carrier used as the labeling substance is especially preferably gold particles because gold particles are simple to detect and do not easily coagulate and because nonspecific color development is unlikely to occur. The average particle diameter of the gold particles is, for example, from 10 nm to 250 nm, and preferably from 35 nm to 120 nm. The average particle diameter can be calculated by measuring the projected area circle equivalent diameters of 100 particles at random using projected pictures taken with a transmission electron microscope (TEM: manufactured by JEOL Ltd., JEM-2010) and calculating from the average.

The amount of the gold particles contained in the labeling substance retaining part is, per unit area of the labeling substance retaining part, usually from 0.006 µg/cm² to 0.42 µg/cm², preferably from 0.01 µg/cm² to 0.3 µg/cm², and more preferably from 0.01 µg/cm² to 0.2 µg/cm². This is because, by determining the amount in the range, the labeled particles can be developed while the particles are dispersed, and the recognition sites for the antibody are not inhibited, resulting in an increase in the sensitivity.

The chromatography medium part (3) is a part for development in the immunochromatography analysis device of the invention. The chromatography medium part (3) is an inert membrane composed of a fine porous substance which causes a capillary phenomenon.

For example, membranes made of nitrocellulose (also called nitrocellulose membranes below) and membranes made of cellulose acetate (also called cellulose acetate membranes below) are preferable, and nitrocellulose membranes are further preferable, because the membranes do not have the property of reacting with the detection reagent or the immobilizing reagent used for the immunochromatography analysis device of the invention or with the substance to be detected or the like and because the effects of the invention are enhanced. In this regard, cellulose membranes, nylon membranes and porous plastic clothes (polyethylene or polypropylene) can also be used.

The nitrocellulose membranes may be any nitrocellulose membranes as long as the membranes mainly contain nitrocellulose, and membranes containing nitrocellulose as the main material, such as a pure product or a nitrocellulose-mixed product, can be used.

The nitrocellulose membranes can contain also a substance which further enhances the capillary phenomenon. The substance is preferably a substance which weakens the surface tension of the membrane surface and attains hydrophilicity.

For example, a substance which has amphipathic action, such as saccharides, derivatives of amino acids, fatty acid esters, various synthetic surfactants or alcohols, and which does not affect the movement of the substance to be detected and does not affect the color development of the labeling substance (such as colloidal gold particles) is preferable.

The nitrocellulose membranes are porous and cause a capillary phenomenon. The indicator of the capillary phenomenon can be confirmed by measuring the speed of water absorption (water absorption time: capillary flow time). The speed of water absorption affects the detection sensitivity and the test time.

The form and the size of the chromatography medium part (3), which is typically any of the nitrocellulose membranes or the cellulose acetate membranes described above, are not particularly limited and may be any form or any size as long as they are appropriate for the actual operation and for the observation of the reaction results.

In order to further make the operation simpler, a support composed of plastic or the like is preferably provided on the back surface of the chromatography medium part (3). The properties and state of the support are not particularly limited, but when the measurement results are observed by a visual evaluation, the support is preferably a support having a color which is not similar to the color achieved by the labeling substance, and the support is usually preferably colorless or white.

The detection part (4) is formed on the chromatography medium part (3). Namely, an antibody which recognizes α-gliadin as the substance to be detected is immobilized at any position on the chromatography medium part (3). The second antibody can be immobilized according to a general method.

The amount of the antibody which recognizes α-gliadin (also called the second antibody below) in the detection part (4) is usually from 0.01 µg/device to 0.8 µg/device, preferably from 0.03 µg/device to 0.6 µg/device, and more preferably from 0.1 µg/device to 0.5 µg/device.

Moreover, the amount of the second antibody per unit area of the detection part (4) is usually from 0.025 µg/cm² to 3 µg/cm², preferably from 0.05 µg/cm² to 2 µg/cm², and more preferably from 0.1 µg/cm² to 1 µg/cm²,

In order to prevent the deterioration of the analysis accuracy due to nonspecific adsorption on the chromatography medium part (3), the chromatography medium part (3) can be subjected to blocking treatment by a known method according to the need.

For the blocking treatment, in general, a protein such as bovine serum albumin, skim milk, casein or gelatin is preferably used. After the blocking treatment, the chromatography medium part (3) may be washed with one or a combination of two or more of surfactants such as polyethylene glycol sorbitan monolaurate (for example, Tween 20: manufactured by Wako Pure Chemical Industries, Ltd.), polyoxyethylene octylphenyl ether (for example, Triton X-100: manufactured by Wako Pure Chemical Industries, Ltd.) or sodium dodecyl sulfate (for example, SDS: manufactured by Wako Pure Chemical Industries, Ltd.) according to the need.

The absorption part (5) is provided at the end of the chromatography medium part (3) to absorb liquid such as the analyte and the development solution which have passed through the detection part (4). For the absorption part (5) in the immunochromatography analysis device of the invention, for example, glass fibers, pulp, cellulose fibers, these nonwoven clothes to which a polymer such as acrylic polymers and a hydrophilic agent having an ethylene oxide group or the like have been added or the like can be used. Glass fibers are preferable. When glass fibers are used for the absorption part (5), the backflow of the sample solution can be reduced considerably.

The backing sheet (6) is a base material. One surface thereof has adhesiveness because an adhesive is added on the surface or an adhesive tape is attached, and the sample addition part (1), the labeling substance retaining part (2), the chromatography medium part (3), the detection part (4) and the absorption part (5) are partially or entirely closely adhered and provided on the adhesive surface. The base material is not particularly limited as long as the backing sheet (6) is impermeable or moisture impermeable with respect to the sample solution due to the adhesive.

The immunochromatography analysis device produced in the above manner is usually subjected to drying treatment before being finished as a product. The drying temperature is, for example, 20 to 50°C, and the drying time is 0.5 to 1 hour.

In the immunochromatography analysis device of the invention, the antibodies which recognize α-gliadin and which are contained in the labeling substance retaining part and in the detection part are antibodies which recognize at least one amino acid sequence of the five amino acid sequences of SEQ ID NOs: 2 to 6 which are present in the whole amino acid sequence of α-gliadin. When antibodies which recognize at least one of the amino acid sequences are used, a cross-reaction with another antigen can be reduced more, and gliadin can be detected more specifically.

The antibody contained in the labeling substance retaining part (the first antibody) and the antibody contained in the detection part (the second antibody) are the same antibody. When the same antibody is used as the first antibody and the second antibody, various conditions (for example, temperature conditions) can be made the same for both antibodies. As a result, variations, such as enhanced or weakened reactivity of only one of the antibodies to the antigen, can be prevented. Moreover, when the reactions of the antibodies with another antigen (cross-reactions) are made the same, the occurrence of a false positive reaction under various conditions can be reduced.

In the immunochromatography analysis device of the invention, the antibodies which recognize α-gliadin and which are contained in the labeling substance retaining part and in the detection part are antibodies which recognize at least one amino acid sequence of the five amino acid sequences of SEQ ID NOs: 2 to 6 which are present in the whole amino acid sequence of α-gliadin and the antibody contained in the labeling substance retaining part (the first antibody) and the antibody contained in the detection part (the second antibody) are the same antibody.

When the antibody contained in the labeling substance retaining part and the antibody contained in the detection part are the same antibody and when the antibody is an antibody which recognizes at least one amino acid sequence of the five amino acid sequences of SEQ ID NOs: 2 to 6, it is presumed that at least the following two cases are the embodiments in which the antibody recognizes the amino acid sequence of α-gliadin.

(1) A case where the primary structure of a repeated sequence present in the five amino acid sequences of SEQ ID NOs: 2 to 6 is recognized
(2) A case where a three-dimensional structure common to the five amino acid sequences of SEQ ID NOs: 2 to 6 is recognized

The two embodiments are explained below.

(1) A case where the primary structure of a repeated sequence present in the five amino acid sequences of SEQ ID NOs: 2 to 6 is recognized

The repeated sequences shown below are present in the five amino acid sequences of SEQ ID NOs: 2 to 6.
SEQ ID NO: 3 and SEQ ID NO: 4: PQQPYPQ
SEQ ID NO: 3 and SEQ ID NO: 4: PYPQPQP
SEQ ID NO: 3 (2 sites): PQPQPFP
SEQ ID NO: 5 (2 sites) and SEQ ID NO: 6: PSSQVSFQ

When the antibody of the invention recognizes the primary structure of any of the repeated amino acid sequences, it is presumed that a sandwich structure described below is formed by conducting an analysis using the immunochromatography analysis device of the invention even when the antibody contained in the labeling substance retaining part and the antibody contained in the detection part are the same antibody and that the detection of gliadin to be detected becomes thus possible.

Namely, when the antibody is contained in the labeling substance retaining part and in the detection part of the immunochromatography analysis device of the invention, the antibody held in the labeling substance retaining part (the first antibody) firstly binds to any of the repeated sequences present in α-gliadin. Then, the antibody immobilized on the detection part (the second antibody) binds to the same repeated sequence which is present at a different site from the site to which the first antibody held in the labeling substance retaining part is bound. Thus, a sandwich structure in which α-gliadin is sandwiched between the first antibody and the second antibody is formed, and gliadin is detected.

In this case, the antibody can recognize more than one site of the amino acid sequence of α-gliadin because the antibody recognizes any of the repeated sequences. Also, the detection sensitivity to gliadin can be improved, and the cross-reactivity can be reduced.

In particular, because the amino acid sequence of SEQ ID NO: 3 has many repeated sequences, the antibody is preferably an antibody which recognizes the amino acid sequence of SEQ ID NO: 3. An antibody which recognizes the amino acid sequence of SEQ ID NO: 3 can recognize more sites in the amino acid sequence of α-gliadin, and the detection sensitivity to α-gliadin can be improved.

### (2) A case where a three-dimensional structure common to the five amino acid sequences of SEQ ID NOs: 2 to 6 is recognized

When there are highly homologous amino acid sequence sites in the five amino acid sequences of SEQ ID NOs: 2 to 6 in α-gliadin, the highly homologous amino acid sequence sites have highly similar chemical properties of the amino acid sequences and may have same or similar three-dimensional structures. In this case, the three-dimensional structure of the entire α-gliadin protein has two or more sites having same or similar three-dimensional structures.

When the antibody in the invention recognizes the common three-dimensional structure of the amino acid sequence, a sandwich structure described below is formed by conducting an analysis using the immunochromatography analysis device of the invention even when the antibody contained in the labeling substance retaining part and the antibody contained in the detection part are a same antibody, and the detection of gliadin to be detected becomes thus possible.

Namely, when the antibody is contained in the labeling substance retaining part and in the detection part of the immunochromatography analysis device of the invention, the antibody held in the labeling substance retaining part (the first antibody) first binds to any of the common three-dimensional structures present in α-gliadin. Then, the antibody immobilized on the detection part (the second antibody) binds to the same three-dimensional structure that is present at a different site from the site to which the first antibody held in the labeling substance retaining part is bound. Thus, a sandwich structure in which α-gliadin is sandwiched between the first antibody and the second antibody is formed, and gliadin is detected.

Also in this case, the antibody can recognize more than one site of the three-dimensional structure of α-gliadin because the antibody recognizes the same plural three-dimensional structures. Also, the detection sensitivity to gliadin can be improved, and the cross-reactivity can be reduced.

### <Immunochromatography Analysis Kit>

The immunochromatography analysis kit of the invention includes the immunochromatography analysis device and an analyte dilution solution for diluting and developing an analyte.

In the immunochromatography analysis kit of the invention, the analyte dilution solution can be used also as a development solution. Water is usually used as a solvent of the analyte dilution solution, and a kind or two or more kinds of the following materials may be added to the solvent: a buffer solution; a salt; a nonionic surfactant; a protein, a polymer compound (such as PVP), an ionic surfactant or a polyanion for, for example, promoting the antigen-antibody reaction or inhibiting a nonspecific reaction; an antibacterial agent; a chelating agent; and the like.

When the analyte dilution solution is used as a development solution, the analyte and the development solution can be mixed in advance and then supplied/added to the sample addition part for development, or the development solution may be supplied/added to the sample addition part for development after supplying/adding the analyte to the sample addition part in advance.

### <Immunochromatography Analysis Method>

The immunochromatography analysis method of the invention includes the following steps (1) to (4), and the substance to be detected, gliadin, contained in an analyte is detected using the immunochromatography analysis device of the invention.
(1) A step of adding an analyte-containing solution obtained by diluting the analyte with an analyte dilution solution to the sample addition part
(2) a step of allowing the first antibody to bind to any of the repeated sequences present in α-gliadin,
(3) a step of developing the analyte and the antibody as a mobile phase on the chromatography medium part, and
(4) a step of detecting α-gliadin in the developed mobile phase with a second antibody that binds to the same repeated sequence which is present at a different site from the site to which the first antibody bound.

Each step is explained below.

(1) A step of adding an analyte-containing solution obtained by diluting the analyte with an analyte dilution solution to the sample addition part

In the step (1), first, an analyte-containing solution is preferably obtained by adjusting or diluting the analyte with an analyte dilution solution to a concentration at which the analyte moves smoothly in the device without deteriorating the measurement accuracy. Those described above can be used as the analyte dilution solution.

Secondly, a certain amount (usually 0.1 to 2 ml) of the analyte-containing solution is added onto the sample addition part (1). When the analyte-containing solution is added, the analyte-containing solution starts to move in the sample addition part (1).

The analyte used in the invention is an analyte which may contain gliadin, which is the substance to be detected. Examples include foods, regardless of being processed or not, which are obtained with using wheat as a raw material such as wheat flour, bread, pasta and tempura.

The analyte sample can be prepared by any known method in addition to the methods described in the Examples. A method for preparing the analyte sample in the case where the analyte is a food is explained below with an example.
1. A food obtained with using wheat as a raw material is pulverized or made into a paste in a homogeneous state with a mill. As the mill, a food cutter (MK-K48; manufactured by Panasonic Corporation), a millser (IFM700G; manufactured by Iwatani Corporation) or the like can be used. With respect to the purpose of the homogenization, it is necessary to make the food into a homogeneous state because a specific raw material is highly likely to be contained unevenly in the food. When the food is a liquid food (such as juice) or a powder food (such as wheat flour), however, the homogenization (pulverization) is not necessary.
2. Proteins are extracted from the pulverized food. For example, by adding 38 mL of the analyte dilution solution to 2 g of the pulverized sample and repeating an extraction operation for 30 seconds with a homogenizer or the like three times, proteins are extracted from the food.
3. The extract solution is centrifuged, and large insoluble components in the extract solution are removed. The supernatant after the centrifugation is filtered with using filter paper, and the obtained filtrate is diluted 10-fold with the analyte dilution solution. The analyte sample can be thus prepared.

(2) A step of allowing the first antibody to bind to any of the repeated sequences present in α-gliadin The step (2) is a step for transferring the analyte-containing solution added to the sample addition part in the step (1) to the labeling substance retaining part (2) and allowing the antibody recognizing α-gliadin (the first antibody) to which the labeling substance is bound and which is held in the labeling substance retaining part to recognize gliadin, which is the substance to be detected, in the analyte. Those described above can be used as the labeling substance.

(3) A step of developing the analyte and the antibody as a mobile phase on the chromatography medium part

The step (3) is a step in which, after gliadin, which is the substance to be detected, has been recognized by the antibody recognizing α-gliadin (the first antibody) to which the labeling substance is bound in the labeling substance retaining part in the step (2), the analyte and the first antibody are caused to pass through on the chromatography medium part as a mobile phase.

(4) A step of detecting gliadin in the developed mobile phase with a second antibody that binds to the same repeated sequence which is present at a different site from the site to which the first antibody bound.

The step (4) is a step in which gliadin in the analyte that has passed through on the chromatography medium part as the mobile phase specifically reacts and binds by a specific antigen-antibody binding reaction in a manner that gliadin is sandwiched between the antibody recognizing α-gliadin (the second antibody) that is held in, namely, immobilized on, the detection part and the antibody recognizing α-gliadin (the first antibody) to which the labeling substance has bound in the step (2), resulting in the coloration of the detection part.

When gliadin, which is the substance to be detected, is absent, the labeling reagent dissolved in the water content of the sample does not cause the specific binding reaction even when the labeling reagent passes through the detection part on the chromatography medium part, and thus the detection part is not colored.

At the end, the water content of the analyte-containing solution moves to the absorption part (5).

### Examples

Although the invention is further explained below with Examples, the invention is not limited to the following examples.

### <Test Example 1>

Two different types of antibody recognizing α-gliadin were prepared in this test, and the test was conducted to confirm that the antibodies were antibodies recognizing at least one amino acid sequence of the five amino acid sequences of SEQ ID NOs: 2 to 6 which are present in the whole amino acid sequence of α-gliadin. The antibodies used are two types, namely an antibody A (XGY06: manufactured by XEMA) and an antibody B (XGY10: manufactured by XEMA).

First, peptides 1 to 5 having the five amino acid sequences of SEQ ID NOs: 2 to 6 which are present in the whole amino acid sequence of α-gliadin were produced by solid-phase peptide synthesis, which is a general method for chemically synthesizing a peptide.
Peptide 1: QPQNPSQQQPQEQVPLVQQQQ (SEQ ID NO: 2)
Peptide 2: PQQPYPQPQPFPSQQPYLQLQPFPQPQPFP (SEQ ID NO: 3)
Peptide 3: PQLPYPQPQSFPPQQPYPQQQPQYLQPQQP (SEQ ID NO: 4)
Peptide 4: PSSQVSFQQPQQQYPSSQVSFQ (SEQ ID NO: 5)
Peptide 5: QYPSSQVSFQPSQLNPQAQGS (SEQ ID NO: 6)

Each peptide was immobilized on a Nunc Immuno modules (manufactured by ThermoFisher Scientific, code 469949) ELISA 96-well plate, and the reaction with each antibody was observed by antigen-immobilized ELISA, which is a general method, by measuring the absorbance with a microplate reader (manufactured by BIORAD).

The following marks were given: + when a signal indicating that the antibody was bound was obtained; ++ when the signal indicating that the antibody was bound was especially stronger than other signals; and - when only a signal equivalent to that of the blank could be obtained. The results are shown in Table 1.

### [Table 1]

**Table 1**

| | Antibody A | Antibody B |
|---|---|---|
| Peptide 1 (SEQ ID NO: 2) | + | - |
| Peptide 2 (SEQ ID NO: 3) | ++ | + |
| Peptide 3 (SEQ ID NO: 4) | + | - |
| Peptide 4 (SEQ ID NO: 5) | - | + |
| Peptide 5 (SEQ ID NO: 6) | - | + |

From the above results, it was found that the antibody A and the antibody B both recognize at least one amino acid sequence of the five amino acid sequences of SEQ ID NOs: 2 to 6 (the peptides 1 to 5). It was also found that the amino acid sequence of SEQ ID NO: 3 (the peptide 2) is recognized by both of the antibody A and the antibody B.

<Test Example 2>

In this test, the immunochromatography analysis kit of the invention was produced, and gliadin was detected using gliadin and foods containing gliadin as analytes.

### [Example 1]

First, an immunochromatography analysis kit composed of an analyte dilution solution and an immunochromatography analysis device including a sample addition part (1), a labeling substance retaining part (2), a chromatography medium part (3) having a detection part (4) and an absorption part (5) was produced.

The antibody contained in the labeling substance retaining part and the antibody contained in the detection part were a same antibody, and the antibody used was the antibody A (XGY06: manufactured by XEMA).

### (1) Production of Sample Addition Part

A nonwoven cloth composed of glass fibers (manufactured by Millipore Corporation: 300 mm × 30 mm) was used as the sample addition part.

### (2) Production of Labeling substance retaining part

To 0.5 ml of a colloidal gold suspension (manufactured by Tanaka Kikinzoku Kogyo K.K.: LC 40 nm), 0.1 ml of the antibody A (XGY06: manufactured by XEMA) which had been diluted to a concentration of 0.05 mg/ml with a phosphate buffer (pH 7.4) was added, and the mixture was left to stand still at room temperature for 10 minutes.

Next, 0.1 ml of a phosphate buffer (pH 7.4) containing 1 mass% bovine serum albumin (BSA) was added, and the mixture was further left to stand still at room temperature for 10 minutes. Then, after stirring thoroughly, the mixture was centrifuged at 8000× g for 15 minutes, and the supernatant was removed. Then, 0.1 ml of a phosphate buffer (pH 7.4) containing 1 mass% BSA was added. A labeling substance solution was produced by the above procedures.

A solution obtained by adding 300 µL of a 10 mass% aqueous trehalose solution and 1.8 mL of distilled water to 300 µL of the labeling substance solution produced above was evenly added to a 12 mm × 300 mm glass fiber pad (manufactured by Millipore Corporation) and then dried with a vacuum dryer, and the labeling substance retaining part was thus produced.

### (3) Production of Chromatography Medium Part and Detection Part

A sheet composed of nitrocellulose (manufactured by Millipore Corporation, product name: HF120, 300 mm × 25 mm) was used as a membrane.

Next, 150 µL of a solution obtained by diluting the antibody A (XGY06: manufactured by XEMA) to a concentration of 1.0 mg/ml with a phosphate buffer (pH 7.4) containing 5 mass% isopropyl alcohol was added to a detection part (a detection line) on the dried membrane in a line with a width of 1 mm using a dispenser for immunochromatography "XYZ3050" (manufactured by BIODOT) at an amount of 1 µL/mm (25 µL per sheet).

Moreover, to check whether the gold nanoparticle labeling reagent has been developed or not and to check the development speed, a solution obtained by diluting goat-derived antiserum having a broad affinity range with the gold nanoparticle labeling reagent with a phosphate buffer (pH 7.4) was added to a control part (a control line) in the downstream of the detection part. Then, by drying at 50°C for 30 minutes and drying at room temperature overnight, the chromatography medium part and the detection part were produced.

### (4) Production of Immunochromatography Analysis Device

Next, the sample addition part, the labeling substance retaining part, the chromatography medium part having the detection part and a nonwoven cloth made of glass fibers as an absorption part for absorbing the developed sample and the labeling substance were attached one by one to a base material composed of a backing sheet. Then, the obtained product was cut with a width of 5 mm with a cutter, and the immunochromatography analysis device was thus obtained. The length of the labeling substance retaining part in the direction of sample development was adjusted to 12 mm.

### (5) Preparation of Analyte Dilution Solution

A 50 mM HEPES buffer (pH 7.5) containing 1 mass% nonionic surfactant (a 1:1 mixture of NP-40 manufactured by Nacalai Tesque, Inc. and Nonidet MN-811 manufactured by NOF Corporation) was prepared and used as the analyte dilution solution for diluting an analyte.

### (6) Measurement

By using the immunochromatography analysis kit produced above, the presence or absence of gliadin to be detected in an analyte was measured by the following method.

The analyte was purified gliadin (manufactured by XEMA). The purified gliadin was diluted with the analyte dilution solution to 2 ng/mL, and an analyte-containing solution was thus prepared.

The prepared analyte-containing solution in an amount of 150 µL was added to the sample addition part of the immunochromatography analysis device and developed, and a visual evaluation was made after 15 minutes. Any of the following marks was given: "+" when the red test line could be visually observed; "++" when the red line could be visually observed clearly; "±" when a light red line could be visually observed; and "-" when the red line could not be visually observed. The results are shown in Table 2.

### [Example 2]

An immunochromatography analysis kit was produced and measurement was conducted in the same manners as in Example 1 except that the antibody B (XGY10: manufactured by XEMA) was used as both of the antibody contained in the labeling substance retaining part and the antibody contained in the detection part. The results are shown in Table 2.

### [Example 3]

An immunochromatography analysis kit was produced and measurement was conducted in the same manners as in Example 1 except that the antibody contained in the labeling substance retaining part was the antibody A (XGY06: manufactured by XEMA) and that the antibody contained in the detection part was the antibody B (XGY10: manufactured by XEMA). The results are shown in Table 2.

### [Example 4]

An immunochromatography analysis kit was produced and measurement was conducted in the same manners as in Example 1 except that the antibody contained in the labeling substance retaining part was the antibody B (XGY10: manufactured by XEMA) and that the antibody contained in the detection part was the antibody A (XGY06: manufactured by XEMA). The results are shown in Table 2.

### [Table 2]

**Table 2**

| | Example 1 | Example 2 | Example 3 | Example 4 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | ++ | ++ | + | + |

As a result of the test, gliadin could be detected when either or both of the antibody A and the antibody B were used. In particular, gliadin could be detected with high sensitivity in Examples 1 and 2, in which a same antibody was used for the labeling substance retaining part and for the detection part.

### [Example 5]

An immunochromatography analysis kit was produced and measurement was conducted in the same manners as in Example 1 except that the analyte was wheat flour. The wheat flour as the analyte was treated as follows, and an analyte-containing solution was prepared. Namely, 0.1 g of commercial wheat flour (manufactured by Nisshin Flour Milling Inc.) was taken, and 1 mL of 70% ethanol was added. Then, insoluble components were precipitated by centrifugation, and the supernatant was used as a wheat flour extract. The extract was diluted 100-fold with the analyte dilution solution, and the analyte-containing solution was thus prepared.

The results are shown in Table 3.

### [Example 6]

An immunochromatography analysis kit was produced and measurement was conducted in the same manners as in Example 5 except that the antibody B (XGY10: manufactured by XEMA) was used as both of the antibody contained in the labeling substance retaining part and the antibody contained in the detection part. The results are shown in Table 3.

### [Example 7]

An immunochromatography analysis kit was produced and measurement was conducted in the same manners as in Example 5 except that the antibody contained in the labeling substance retaining part was the antibody A (XGY06: manufactured by XEMA) and that the antibody contained in the detection part was the antibody B (XGY10: manufactured by XEMA). The results are shown in Table 3.

### [Example 8]

An immunochromatography analysis kit was produced and measurement was conducted in the same manners as in Example 5 except that the antibody contained in the labeling substance retaining part was the antibody B (XGY10: manufactured by XEMA) and that the antibody contained in the detection part was the antibody A (XGY06: manufactured by XEMA). The results are shown in Table 3.

### [Table 3]

**Table 3**

| | Example 5 | Example 6 | Example 7 | Example 8 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | ++ | ++ | ++ | + |

As a result of the test, gliadin in the wheat flour, which was the analyte, could be detected when either or both of the antibody A and the antibody B were used.

### [Example 9]

An immunochromatography analysis kit was produced and measurement was conducted in the same manners as in Example 1 except that the analyte was bread (manufactured by Fuji Baking Co., Ltd., product name: *Komugi no Choushoku).* The bread as the analyte was treated as follows, and an analyte-containing solution was prepared. Namely, 0.1 g of commercial bread was taken, and 1 mL of 70% ethanol was added. Then, insoluble components were precipitated by centrifugation, and the supernatant was used as a bread extract. The extract was diluted 100-fold with the analyte dilution solution, and the analyte-containing solution was thus prepared.

The results are shown in Table 4.

### [Example 10]

An immunochromatography analysis kit was produced and measurement was conducted in the same manners as in Example 9 except that the antibody B (XGY10: manufactured by XEMA) was used as both of the antibody contained in the labeling substance retaining part and the antibody contained in the detection part. The results are shown in Table 4.

### [Example 11]

An immunochromatography analysis kit was produced and measurement was conducted in the same manners as in Example 9 except that the antibody contained in the labeling substance retaining part was the antibody A (XGY06: manufactured by XEMA) and that the antibody contained in the detection part was the antibody B (XGY10: manufactured by XEMA). The results are shown in Table 4.

### [Example 12]

An immunochromatography analysis kit was produced and measurement was conducted in the same manners as in Example 9 except that the antibody contained in the labeling substance retaining part was the antibody B (XGY10: manufactured by XEMA) and that the antibody contained in the detection part was the antibody A (XGY06: manufactured by XEMA). The results are shown in Table 4.

### [Table 4]

**Table 4**

| | Example 9 | Example 10 | Example 11 | Example 12 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | ++ | ++ | + | + |

As a result of the test, gliadin in the bread, which was the analyte, could be detected when either or both of the antibody A and the antibody B were used. In particular, the bread (gliadin) could be detected with high sensitivity in Examples 9 and 10, in which a same antibody was used for the labeling substance retaining part and for the detection part.

### <Test Example 3>

### [Example 13]

The immunochromatography analysis kit of Example 1 (Test Example 2), in which the antibody A (XGY06: manufactured by XEMA) was contained both in the labeling substance retaining part and in the detection part, was used, and measurement was conducted in the same manners as in Example 1 but using purified corn protein (purified zein below), commercial buckwheat flour, commercial common beans and commercial salmon flakes as analytes. The analyte-containing solution of purified zein was produced in a manner that the concentration thereof became 2 ng/mL.

With respect to buckwheat flour, common beans and salmon flakes, commercial buckwheat flour, common beans and salmon flakes each in an amount of 0.1 g were taken, and 1 mL of 70% ethanol was added. Then, insoluble components were precipitated by centrifugation, and the supernatants were used as the extracts. The extracts were diluted 100-fold with the analyte dilution solution, and analyte-containing solutions were thus prepared.

The results are shown in Table 5.

### [Comparative Example 1]

Measurement was conducted in the same manners as in Example 13 except that a commercial immunochromatography kit for gliadin detection (manufactured by NH Foods Ltd., FASTKIT Slim) was used. The results are shown in Table 5.

### [Table 5]

**Table 5**

| | Example 13 | Comparative Example 1 |
|---|---|---|
| Purified zein | - | ± |
| Buckwheat flour | - | ± |
| Common beans | - | + |
| Salmon flakes | - | + |

As a result of the test, none of the cross-reactions with the analytes, purified zein, buckwheat flour, common beans and salmon flakes, was observed in the immunochromatography analysis kit of the invention. On the other hand, the cross-reactions with all the analytes were observed in the commercial immunochromatography kit for gliadin detection of Comparative Example 1. Accordingly, it was found that cross-reactions with antigens other than gliadin are inhibited in the immunochromatography analysis kit of the invention as compared to the commercial immunochromatography kit for detecting gliadin.

### <Test Example 4>

In the immunochromatography analysis kit of the invention, the cross-reactions with some antigens other than gliadin were not observed with the analyte concentrations of Test Example 3. Thus, in this test, by increasing the concentrations of the analytes used in Test Example 3 (purified zein, buckwheat flour, common beans and salmon flakes) 5000-fold, the most suitable combinations of the antibodies, with respect to the cross-reactivities with antigens other than gliadin, were investigated.

### [Reference Examples 1 to 4]

A test was conducted in the same manners as in Test Example 3 (Example 13) except that the concentration of purified zein as the analyte in the analyte-containing solution was 10 µg/mL, which was 5000 times higher than the concentration in Test Example 3, and that the combinations of the antibodies contained in the labeling substance retaining part and in the detection part were as shown in Table 6. The results are shown in Table 6.

### [Table 6]

**Table 6**

| | Reference Example 1 | Reference Example 2 | Reference Example 3 | Reference Example 4 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | - | - | ± | - |

### [Reference Examples 5 to 8]

A test was conducted in the same manners as in Test Example 3 (Example 13) except that the analyte-containing solution was prepared in a manner that the concentration of buckwheat flour as the analyte became 5000 times higher than the concentration in Test Example 3 (Example 13) and except that the combinations of the antibodies contained in the labeling substance retaining part and in the detection part were as shown in Table 7. The results are shown in Table 7.

### [Table 7]

**Table 7**

| | Reference Example 5 | Reference Example 6 | Reference Example 7 | Reference Example 8 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | ± | ± | + | + |

### [Reference Examples 9 to 12]

A test was conducted in the same manners as in Test Example 3 (Example 13) except that the analyte-containing solution was prepared in a manner that the concentration of common beans as the analyte became 5000 times higher than the concentration in Test Example 3 (Example 13) and except that the combinations of the antibodies contained in the labeling substance retaining part and in the detection part were as shown in Table 8. The results are shown in Table 8.

### [Table 8]

**Table 8**

| | Reference Example 9 | Reference Example 10 | Reference Example 11 | Reference Example 12 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | - | - | + | + |

### [Reference Examples 13 to 16]

A test was conducted in the same manners as in Test Example 3 (Example 13) except that the analyte-containing solution was prepared in a manner that the concentration of salmon flakes as the analyte became 5000 times higher than the concentration in Test Example 3 (Example 13) and except that the combinations of the antibodies contained in the labeling substance retaining part and in the detection part were as shown in Table 9.

The results are shown in Table 9.

### [Table 9]

**Table 9**

| | Reference Example 13 | Reference Example 14 | Reference Example 15 | Reference Example 16 |
|---|---|---|---|---|
| Labeling substance-holding part side | Antibody A | Antibody B | Antibody A | Antibody B |
| Detection part side | Antibody A | Antibody B | Antibody B | Antibody A |
| Evaluation | - | - | ± | ± |

As it can be seen also from the results of Reference Examples 1 to 16, it was found that cross-reactions are less likely to occur in the immunochromatography analysis kits of the invention in which a same antibody is used at the labeling substance retaining part side and at the detection part side as compared with the cases where different antibodies are used at the labeling substance retaining part side and at the detection part side.

The present application is based on a Japanese patent application filed on February 23, 2016 (patent application No. 2016-03241 8).

### Reference Signs List

1 Sample addition part
2 Labeling substance retaining part
3 Chromatography medium part
4 Detection part
5 Absorption part
6 Backing sheet

## Claims

1. An immunochromatography analysis device for detecting gliadin, including a sample addition part, a labeling substance retaining part, a chromatography medium part having a detection part and an absorption part,
wherein the labeling substance retaining part contains a first antibody recognizing α-gliadin and the detection part contains a second antibody recognizing α-gliadin, wherein the first and second antibody are the same, and the first antibody binds to any of the repeated sequences present in α-gliadin and the second antibody binds to the same repeated sequence which is present at a different site from the site to which the first antibody bound;
wherein the first and second antibody are monoclonal antibodies, and the first and second antibody recognize at least one amino acid sequence of the five amino acid sequences of SEQ ID NOs: 2 to 6 which are present in the whole amino acid sequence of α-gliadin;
wherein the amount of antibody in the labelling substance retaining part is from 0.01 µg/device to 1.0 µg/device.

2. An immunochromatography analysis kit comprising the immunochromatography analysis device according to claim 1 and an analyte dilution solution for diluting and developing an analyte.

3. An immunochromatography analysis method for detecting gliadin in an analyte using an immunochromatography analysis device including a sample addition part, a labeling substance retaining part, a chromatography medium part having a detection part and an absorption part, wherein the labeling substance retaining part contains a first antibody recognizing α-gliadin and the detection part contains a second antibody recognizing α-gliadin, and the first and second antibody are the same;
wherein the first and second antibody are monoclonal antibodies, and the first and second antibody recognize at least one amino acid sequence of the five amino acid sequences of SEQ ID NOs: 2 to 6 which are present in the whole amino acid sequence of α-gliadin; and wherein the amount of antibody in the labelling substance retaining part is from 0.01 µg/device to 1.0 µg/device;
wherein the method includes the following steps (1) to (4):
(1) a step of adding an analyte-containing solution obtained by diluting the analyte with an analyte dilution solution to the sample addition part,
(2) a step of allowing the first antibody to bind to any of the repeated sequences present in α-gliadin,
(3) a step of developing the analyte and the antibody as a mobile phase on the chromatography medium part, and
(4) a step of detecting gliadin in the developed mobile phase with a second antibody that binds to the same repeated sequence which is present at a different site from the site to which the first antibody bound.

## Patentansprüche

1. Immunchromatographie-Analysevorrichtung zum Nachweis von Gliadin, die einen Probenzugabeteil, einen Markierungssubstanz-Rückhalteteil, einen Chromatographiemediumteil mit einem Nachweisteil und einem Absorptionsteil umfasst,
wobei der Markierungssubstanz-Rückhalteteil einen ersten Antikörper enthält, der α-Gliadin erkennt, und der Nachweisteil einen zweiten Antikörper enthält, der α-Gliadin erkennt, wobei der erste und zweite Antikörper gleich sind, und der erste Antikörper an eine der in α-Gliadin vorhandenen wiederholten Sequenzen bindet und der zweite Antikörper an die gleiche wiederholte Sequenz bindet, die an einer anderen Stelle vorhanden ist als die Stelle, an die der erste Antikörper gebunden hat;
wobei der erste und zweite Antikörper monoklonale Antikörper sind, und der erste und zweite Antikörper mindestens eine Aminosäuresequenz der fünf Aminosäuresequenzen der SEQ ID Nr. 2 bis 6 erkennen, die in der gesamten Aminosäuresequenz von α-Gliadin vorhanden sind;
wobei die Menge des Antikörpers in dem Markierungssubstanz-Rückhalteteil von 0,01 µg/Vorrichtung bis 1,0 µg/Vorrichtung ist.

2. Immunochromatographie-Analysekit, das die Immunochromatographie-Analysevorrichtung nach Anspruch 1 und eine Analyt-Verdünnungslösung zum Verdünnen und Entwickeln eines Analyten umfasst.

3. Immunochromatographie-Analyseverfahren zum Nachweis von Gliadin in einem Analyten unter Verwendung einer Immunochromatographie-Analysevorrichtung, die einen Probenzugabeteil, einen Markierungssubstanz-Rückhalteteil, einen Chromatographiemediumteil mit einem Nachweisteil und einem Absorptionsteil umfasst, wobei der Markierungssubstanz-Rückhalteteil einen ersten Antikörper enthält, der α-Gliadin erkennt, und der Nachweisteil einen zweiten Antikörper enthält, der α-Gliadin erkennt, und der erste und zweite Antikörper gleich sind;
wobei der erste und zweite Antikörper monoklonale Antikörper sind, und der erste und zweite Antikörper mindestens eine Aminosäuresequenz der fünf Aminosäuresequenzen der SEQ ID Nr. 2 bis 6 erkennen, die in der gesamten Aminosäuresequenz von α-Gliadin vorhanden sind; und wobei die Menge des Antikörpers in dem Markierungssubstanz-Rückhalteteil von 0,01 µg/Vorrichtung bis 1,0 µg/Vorrichtung ist;
wobei das Verfahren die folgenden Schritte (1) bis (4) umfasst:
(1) einen Schritt der Zugabe einer Analyt-enthaltenden Lösung, die durch Verdünnen des Analyten mit einer Analyt-Verdünnungslösung erhalten wird, zu dem Probenzugabeteil,
(2) einen Schritt, bei dem der erste Antikörper an eine der in α-Gliadin vorhandenen wiederholten Sequenzen binden kann,
(3) einen Schritt zur Entwicklung des Analyten und des Antikörpers als eine mobile Phase auf dem Chromatographiemediumteil, und
(4) einen Schritt des Nachweises von Gliadin in der entwickelten mobilen Phase mit einem zweiten Antikörper, der an die gleiche wiederholte Sequenz bindet, die an einer anderen Stelle vorhanden ist als die Stelle, an die der erste Antikörper gebunden hat.

## Revendications

1. Dispositif d'analyse d'immunochromatographie pour détecter la gliadine, incluant une partie d'ajout d'échantillon, une partie de conservation de substance de marquage, une partie de milieu chromatographique présentant une partie de détection et une partie d'absorption,
dans lequel la partie de conservation de substance de marquage contient un premier anticorps reconnaissant l'α-gliadine et la partie de détection contient un second anticorps reconnaissant l'α-gliadine, dans lequel les premier et second anticorps sont identiques, et le premier anticorps se lie à l'une quelconque des séquences répétées présentes dans l'α-gliadine et le second anticorps se lie à la même séquence répétée qui est présente au niveau d'un site différent du site auquel le premier anticorps s'est lié ;
dans lequel les premier et second anticorps sont des anticorps monoclonaux, et les premier et second anticorps reconnaissent au moins une séquence d'acides aminés des cinq séquences d'acides aminés de SEQ ID NOs : 2 à 6 qui sont présentes dans la séquence d'acides aminés entière de l'α-gliadine ;
dans lequel la quantité d'anticorps dans la partie de conservation de substance de marquage est de 0,01 µg/dispositif à 1,0 µg/dispositif.

2. Kit d'analyse d'immunochromatographie comprenant le dispositif d'analyse d'immunochromatographie selon la revendication 1 et une solution de dilution d'analyte pour diluer et développer un analyte.

3. Procédé d'analyse d'immunochromatographie pour détecter la gliadine dans un analyte en utilisant un dispositif d'analyse d'immunochromatographie incluant une partie d'ajout d'échantillon, une partie de conservation de substance de marquage, une partie de milieu chromatographique présentant une partie de détection et une partie d'absorption, dans lequel la partie de conservation de substance de marquage contient un premier anticorps reconnaissant l'α-gliadine et la partie de détection contient un second anticorps reconnaissant l'α-gliadine, et les premier et second anticorps sont identiques ;
dans lequel les premier et second anticorps sont des anticorps monoclonaux, et les premier et second anticorps reconnaissent au moins une séquence d'acides aminés des cinq séquences d'acides aminés de SEQ ID NOs : 2 à 6 qui sont présentes dans la séquence d'acides aminés entière de l'α-gliadine ; dans lequel la quantité d'anticorps dans la partie de conservation de substance de marquage est de 0,01 µg/dispositif à 1,0 µg/dispositif ;
dans lequel le procédé inclut les étapes (1) à (4) suivantes :
(1) une étape consistant à ajouter une solution contenant un analyte obtenue en diluant l'analyte avec une solution de dilution d'analyte à la partie d'ajout d'échantillon,
(2) une étape consistant à permettre au premier anticorps de se lier à l'une quelconque des séquences répétées présentes dans l'α-gliadine,
(3) une étape consistant à développer l'analyte et l'anticorps comme une phase mobile sur la partie de milieu chromatographique, et
(4) une étape consistant à détecter la gliadine dans la phase mobile développée avec un second anticorps qui se lie à la même séquence répétée qui est présente au niveau d'un site différent du site auquel le premier anticorps s'est lié.
